# EUROPÄISCHE PATENTSCHRIFT

(11) **EP 2 285 478 B1**
(45) Veröffentlichungstag und Bekanntmachung des Hinweises auf die Patenterteilung: **30.05.2012**
(21) Anmeldenummer: 09753611.4
(22) Anmeldetag: 07.05.2009
(51) Int. Cl.: B01F 15/00, B01F 13/08, B01F 7/16, F16J 15/32, C12M 1/02

(54) **MISCHSYSTEM**
MIXING SYSTEM
SYSTÈME DE MÉLANGE

(30) Priorität: 28.05.2008 DE 102008025508
(43) Veröffentlichungstag der Anmeldung: 23.02.2011
(73) Patentinhaber: Sartorius Stedim Biotech GmbH, 37079 Göttingen (DE)
(72) Erfinder: REIF, Oscar-Werner, 30173 Hannover (DE); VAN DEN BOOGARD, Jürgen, 37127 Dransfeld (DE); GRELLER, Gerhard, 37085 Göttingen (DE); LÜHMANN, Sven, 37154 Northeim (DE); LOHRENGEL, Marco, 37197 Hattorf am Harz (DE)
(86) Internationale Anmeldenummer: PCT/EP2009/003254
(87) Internationale Veröffentlichungsnummer: WO 2009/143956

(56) Entgegenhaltungen:
- DE-B3-102006 022 651
- JP-A- 2006 212 542
- US-A1- 2005 239 199
- US-A1- 2006 280 028
- US-A1- 2007 053 238

## Beschreibung

Die Erfindung betrifft ein Mischsystem für einen Behälter mit flexiblen Wänden.

Einwegbehälter mit flexiblen Wänden werden zunehmend in der Biotechnologie- und Pharmaindustrie eingesetzt, um Flüssigkeiten zu mischen, Feststoffe in Flüssigkeiten zu lösen oder um Zellen und Mikroorganismen zu vermehren. Dabei bieten Einwegbehälter aus Kunststoff den Vorteil, dass sie bereits vom Lieferanten sauber und gegebenenfalls steril angeliefert werden können, wodurch aufwändige Reinigungsprozeduren und Validierungen vor der Inbetriebnahme entfallen.

Zur effektiven Durchmischung von Medien in Behältern mit flexiblen Wänden, zum Einmalgebrauch eignen sich insbesondere rotierende Rührerwellen, die mit Rührelementen wie z.B. Propellern versehen sind. Zum Antrieb solcher Rührerwellen hat es sich bewährt, die Kopplung des Drehmomentes vom Motor auf die Rührerwelle durch eine berührungslose Magnetkupplung zu bewerkstelligen, welche verschleißfrei betrieben werden kann.

In US-Anmeldung 2007/0053238 A1 wird eine Magnetkupplung für eine Rührerwelle beschrieben, bei der sich Antriebskomponenten wie Lager und Magneten außerhalb des Behälterinnenraums befinden. Nachteilig an dieser Ausführung ist jedoch, dass der Behälterinnenraum, der die zu mischenden Medien enthält, nicht gegen die Antriebskomponenten abgedichtet ist. So befindet sich das erste Lager zur Aufnahme der Rührerwelle direkt an der Öffnung zwischen dem Behälterinnenraum und der Magnetkupplung. Durch diese Anordnung können Lager und die auf der Rührerwelle angebrachten Magneten (Antriebsmagneten) mit den zu mischenden Medien in Kontakt kommen und durch Korrosion Schaden nehmen. Umgekehrt ist es möglich, dass Partikel, die aus den Lagern oder von den Magneten stammen, in den Behälterinnenraum gelangen und somit zu einer Kontamination des Mischgutes führen. Dies wiegt umso schwerer, als diese mechanischen Komponenten i.d.R. aus Metallen gefertigt werden und folglich nicht problemlos durch Gammastrahlen sterilisiert werden können.

Die US 2006/280028 A1 beschreibt eine Mischvorrichtung für Behälter mit flexiblen Wänden, bei der der Antrieb eine Rühreinheit aufnimmt, welche über einen Flansch mit der flexiblen Wand verbunden ist und wobei der Mischerschaft durch den Flansch geführt ist. Der Antrieb wird für eine einfachere Montage von einem zu öffnenden und durch eine Tür verschließbaren Gehäuse aufgenommen. D1 weist keine magnetische Antriebsvorrichtung auf, vielmehr wird lediglich eine Motorenaufnahme offenbart. Weiterhin ist das Merkmal, dass der "Antriebsraum (6) von der Kopfplatte (5) und einer an der Kopfplatte (5) befestigten Kappe (7) umschlossen" ist nicht vorhanden.

Die Patentanmeldung US 2005/239199 A1 beschreibt ein Rührertanksystem mit einem flexiblen Behälter, der von einem Stützbehälter aufgenommen wird. Ein Mischerschaft durchdringt die Behälterwand, an der Außen ein Lager mit Dichtungen zur Hindurchführung des Mischerschaftes angebracht ist. An das nach außen gerichtete Ende des das Lager durchdringenden Mischerschafts ist ein Antriebsmotor angeschlossen. Eine magnetische Antriebsvorrichtung, welche direkt an die mit dem flexiblen Behälter verbundene Kopfplatte angeschlossen ist, wird nicht offenbart.

DE 10 2006 022651 B3 betrifft einen Behälter mit flexiblen Wänden und einem Magnetrührer, wobei die Behälterwandung von einer aufblasbaren Struktur stabilisierbar ist, die im aufgeblasenen Zustand einen Teil des Magnetrührers im Innern des Behälters in Rührposition hält. Der Magnetantrieb befindet sich außerhalb des Behälters und ist mit dem Magnetrührerteil im Innern des Behälters nicht verbunden. Es wird nicht eine Rührerwelle, sondern lediglich ein Mischer ohne Schaft offenbart, da ein platzsparendes System beschrieben wird. Weiterhin wird das Merkmal , "wobei der Antriebsraum (6) von der Kopfplatte (5) und einer an der Kopfplatte (5) befestigten Kappe (7) umschlossen" durch eine Aufblasbare Vorrichtung erörtert;

JP 2006 212542 A beschreibt einen organischen Abfallentsorger zur Benutzung bei der Wasseraufbereitung. Ein spezieller Mischer verhindert die Ablagerung von Feststoffen. Ein magnetischer Antrieb wird nicht offenbart.

Die Aufgabe der Erfindung besteht deshalb darin, ein kostengünstiges Mischsystem mit einem Antrieb für einen Einwegbehälter vorzuschlagen, bei dem die Sterilität des Behälterinnenraums gewährleistet ist.

Die Aufgabe wird erfindungsgemäß durch ein Mischsystem gelöst, bestehend aus einem Behälter mit flexiblen Wänden, mindestens mit einer Kopfplatte und einem fluiddichten Antriebsraum, der sich außerhalb des Behälterinnenraums befindet, wobei der Antriebsraum von der Kopfplatte und von einer an der Kopfplatte befestigten Kappe umschlossen ist, einer Mischvorrichtung mit einer aus Kunststoff gefertigten Rührerwelle, an der aus Kunststoff gefertigte Rührelemente befestigt sind, wobei die Rührerwelle vom Behälterinnenraum durch eine Öffnung in der Kopfplatte geführt ist und wobei der im Antriebsraum befindliche Abschnitt der Rührerwelle von einem oder mehreren Lager(n) geführt ist und wobei in die Öffnung der Kopfplatte eine Dichtung eingepasst ist, welche den Rührerschaft umschließt und den Behälterinnenraum gegen den Antriebsraum abdichtet. Am im Antriebsraum befindlichen Abschnitt der Rührerwelle sind Antriebsmagneten angebracht, welche durch magnetische Kräfte mit Rotormagneten eines Rotors außerhalb des Antriebsraums gekoppelt sind, wobei eine durch einen Motor hervorgerufene Rotation des Rotors auf die Rührerwelle übertragbar ist und wobei die Kappe (7), demontierbar ist und die Rührerwelle im Bereich des Antriebsraumes (6) teilbar ausgestaltet ist, wodurch der Abschnitt der Rüherwelle mit den Antriebsmagneten (13) oder dem Motor abnehmbar ist und einer Wiederverwendung oder getrennten Entsorgung zuführbar ist..

Zusätzlich zu den Lagern auf der Antriebsseite kann die Rührerwelle am entgegen gesetzten Ende durch ein Lager in einer Bodenplatte des Behälters aufgenommen sein, wobei es sich zumindest bei einem der erwähnten Lager um ein Gleitlager handeln kann. Die Lager können vollständig aus Kunststoff gefertigt sein.

Die oben erwähnte Dichtung kann aus Polymermaterialien gefertigt und als Wegwerfartikel in das Mischsystem integriert sein. Die Dichtung kann so gestaltet sein, dass sie den flexiblen Behälter dicht gegenüber Gasen, Flüssigkeiten und Kontaminationen abschließt.

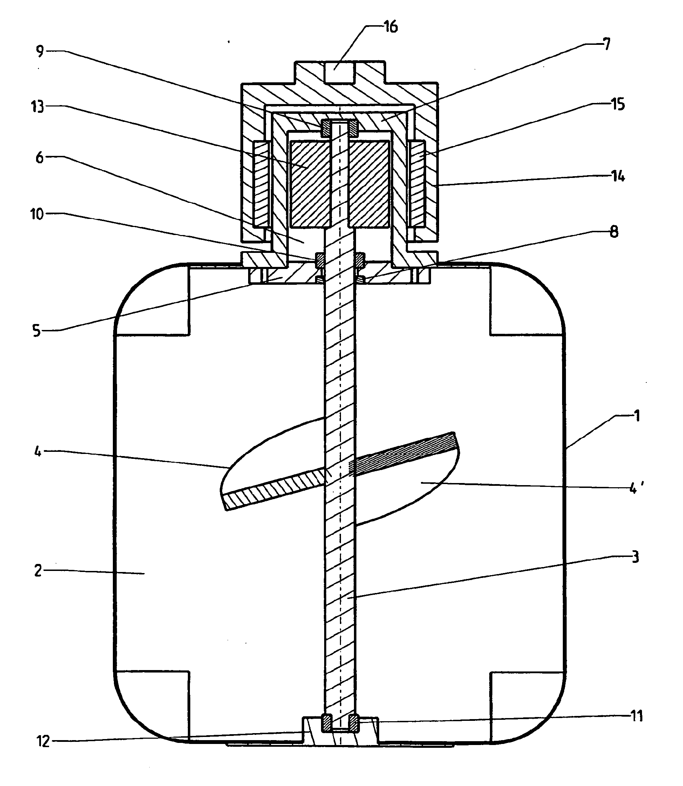

Die Erfindung wird durch die Figur näher erläutert.

Gemäß der Figur umschließt der Behälter **1** einen Behälterinnenraum **2** der zur Aufnahme einer Flüssigkeit dient. Im Behälterinnenraum **2** befindet sich eine Mischvorrichtung, die aus einer aus Kunststoffmaterial gefertigten Rührerwelle **3** und aus Kunststoffmaterial gefertigten Rührelementen **4** und **4'** besteht. Die Rührerwelle **3** durchdringt den Behälter **1** im Bereich der Kopfplatte **5,** und endet in einem Antriebsraum **6,** der von einer Kappe **7** umschlossen ist. In die Kopfplatte **5** ist eine Dichtung **8** eingelassen, die die Rührerwelle **3** umschließt und die den Behälterinnenraum **2** vom Antriebsraum **6** separiert und gegen den Durchgang von Fluiden oder Partikeln abdichtet. Die Rührerwelle **3** wird an ihrem oberen Ende durch ein erstes Gleitlager **9** aufgenommen, das mit der Kappe **7** verbunden ist. Außerdem wird die Rührerwelle **3** durch ein zweites Gleitlager **10** gelagert, dass in die Kopfplatte **5** eingepasst ist. Ein drittes Gleitlager **11,** das in eine Bodenplatte **12** eingepasst ist, nimmt die Rührerwelle am unteren Ende auf. Das Drehmoment zum Antrieb der Rührerwelle **3** wird über die Antriebsmagneten **13** aufgenommen, die sich im Antriebsraum 6 befinden und die am oberen Ende der Rührerwelle **3** angebracht sind. Über die Kappe **7** ist ein Rotor **14** gestülpt, an dem die Rotormagneten **15** angebracht sind. Über einen Motoranschluss **16** ist der Rotor **14** mit einem Motor verbunden. Wird der Rotor **14** durch den Motor in Rotation versetzt, so überträgt sich ein Drehmoment von den Rotormagneten **15** auf die Antriebsmagneten **13**, die wiederum die Rührerwelle **3** mit den Rührelementen **4** und **4**' in Bewegung versetzen.

## Patentansprüche

1. Mischsystem bestehend aus
- einem Behälter mit flexiblen Wänden (1), mindestens mit einer Kopfplatte (5) und
- einem fluiddichten Antriebsraum (6), der sich außerhalb des Behälterinnenraums (2) befindet, wobei der Antriebsraum (6) von der Kopfplatte (5) und einer an der Kopfplatte (5) befestigten Kappe (7) umschlossen ist,
- einer Mischvorrichtung mit einer aus Kunststoff gefertigten Rührerwelle (3), an der aus Kunststoff gefertigte Rührelemente (4, 4') befestigt sind, wobei die Rührerwelle (3) vom Behälterinnenraum (2) durch eine Öffnung in der Kopfplatte (5) geführt ist und wobei der im Antriebsraum (6) befindliche Abschnitt der Rührerwelle (3) von einem oder mehreren Lager(n) (9, 10) geführt ist und wobei in die Öffnung der Kopfplatte eine Dichtung (8) eingepasst ist, welche den Rührerschaft (3) umschließt und den Behälterinnenraum (2) gegen den Antriebsraum (6) abdichtet und wobei an dem im Antriebsraum (6) befindlichen Abschnitt der Rührerwelle Antriebsmagneten (13) angebracht sind, welche durch magnetische Kräfte mit Rotormagneten (15) eines Rotors (14) außerhalb des Antriebsraums (6) gekoppelt sind, wobei eine durch einen Motor hervorgerufene Rotation des Rotors (14) auf die Rührerwelle (3) übertragbar ist und wobei die Kappe (7), demontierbar ist und die Rührerwelle im Bereich des Antriebsraumes (6) teilbar ausgestaltet ist, wodurch der Abschnitt der Rühnerwelle mit den Antriebsmagneten (13) abnehmbar ist und einer Wiederverwendung oder getrennten Entsorgung zuführbar ist.

2. Mischsystem nach Anspruch 1, wobei die Rührerwelle (3) zusätzlich zu den Lagern (9, 10) auf der Antriebsseite am entgegen gesetzten Ende durch ein Lager (11) in einer Bodenplatte (12) des Behälters (1) aufgenommen ist.

3. Mischsystem nach Anspruch 1 oder 2, wobei es sich zumindest bei einem der Lager (9, 10, 11) um ein Gleitlager handelt.

4. Mischsystem nach Anspruch 1, 2 oder 3, wobei die Lager (9, 10, 11) vollständig aus Kunststoff gefertigt sind.

5. Mischsystem nach Anspruch 1, wobei die Dichtung (8) aus Polymermaterialien gefertigt ist und als Wegwerfartikel in das Mischsystem integriert ist.

6. Mischsystem nach Anspruch 1, wobei die Dichtung (8) den Behälter (1) dicht gegenüber Gasen, Flüssigkeiten und Kontaminationen abschließt.

## Claims

1. Mixer system consisting of
- a container with flexible walls (1), at least with a head plate (5) and
- a fluid-tight drive chamber (6), which is disposed outside the container interior space (2), wherein the drive chamber (6) is enclosed by the head plate (5) and a cap (7) fastened to the head plate (5),
- a mixing device with an agitator shaft (3), which is made of plastics material and to which agitator elements (4, 4') made of plastics material are attached, wherein the agitator shaft (3) is led from the container interior space (2) through an opening in the head plate (5) and wherein the section of the agitator shaft (3) located in the drive chamber (6) is guided by one or more bearings (9, 10) and wherein a seal (8), which surrounds the agitator shaft (3) and seals the container interior space (2) relative to the drive chamber (6), is fitted in the opening of the head plate and wherein mounted at the section of the agitator shaft located in the drive chamber (6) are drive magnets (13) which are coupled by magnetic forces with rotor magnets (15) of a rotor (14) outside the drive chamber (6), wherein a rotation of the rotor (14) produced by a motor is transmissible to the agitator shaft (3) and wherein the cap (7) is demountable and the agitator shaft is constructed to be separable in the region of the drive chamber (6), whereby the section of the agitator shaft together with the drive magnets (13) is removable and can be supplied for re-use or separate disposal.

2. Mixer system according to claim 1, wherein the agitator shaft (3) is received, additionally to the bearings (9, 10) on the drive side, at the opposite end by a bearing (11) in a base plate (12) of the container (1).

3. Mixer system according to claim 1 or 2, wherein at least one of the bearings (9, 10, 11) is a slide bearing.

4. Mixer system according to claim 1, 2 or 3, wherein the bearings (9, 10, 11) are made entirely of plastics material.

5. Mixer system according to claim 1, wherein the seal (8) is made of polymer materials and is integrated in the mixer system as a disposable article.

6. Mixer system according to claim 1, wherein the seal (8) sealably closes the container (1) relative to gases, liquid and contaminants.

## Revendications

1. Système de mélange constitué
- d'un récipient comprenant des parois flexibles (1), comprenant au moins un plateau de tête (5) et
- d'un espace de commande (6) étanche aux fluides qui se situe en dehors de l'espace intérieur du récipient (2), l'espace de commande (6) étant entouré du plateau de tête (5) et d'un couvercle (7) fixé sur le plateau de tête (5),
- d'un dispositif de mélange comprenant un arbre d'agitation (3) réalisé en matière synthétique sur lequel sont fixés des éléments d'agitation (4, 4') constitués en matière synthétique, l'arbre d'agitation (3) étant mis en oeuvre à partir de l'espace interne du récipient (2) par une ouverture dans le plateau de tête (5) et la partie de l'arbre d'agitation (3) se trouvant dans l'espace de commande étant guidée par un ou plusieurs roulements (9, 10) et un joint (8) étant adapté à l'ouverture du plateau de tête, joint qui entoure la tige d'agitateur (3) et étanchéifie l'espace intérieur du récipient (2) vis-à-vis de l'espace de commande (6), et des aimants d'entraînement (13) étant rapportés sur la partie de l'arbre d'agitation se trouvant dans l'espace de commande (6), aimants qui sont couplés avec des aimants de rotor (15) d'un rotor (14) à l'extérieur de l'espace de commande (6) par l'intermédiaire de forces magnétiques, une rotation du rotor (14) provoquée par un moteur pouvant être transmise à l'arbre d'agitation (3), et le couvercle (7) pouvant être démonté, et l'arbre d'agitation étant conçu de manière dissociable dans la zone de l'espace de commande (6), par ce moyen, la partie de l'arbre d'agitation peut être enlevée avec les aimants d'entraînement et peut être disponible pour un nouvel emploi ou pour une évacuation séparée.

2. Système de mélange selon la revendication 1 où l'arbre d'agitation (3), en plus des roulements (9, 10) sur l'extrémité opposée, du côté moteur, sont disposés sur un plateau de fond (12) du récipient (1) par l'intermédiaire d'un roulement (11).

3. Système de mélange selon les revendications 1 ou 2 où au moins dans le cas d'un roulement (9, 10, 11), il s'agit d'un palier de glissement.

4. Système de mélange selon les revendications 1, 2 ou 3 où les roulements (9, 10, 11) sont totalement fabriqués en matière synthétique.

5. Système de mélange selon la revendication 1 où le joint (8) est fabriqué dans des matériaux polymériques et est intégré dans le système de mélange sous la forme d'un article jetable.

6. Système de mélange selon la revendication 1 où le joint (8) ferme le récipient (1) de manière étanche vis-à-vis de gaz, de liquides et de contaminations.
